# EUROPEAN PATENT APPLICATION

(11) **EP 2 338 477 A1**
(43) Date of publication of application: **29.06.2011**
(21) Application number: 09015508.6
(22) Date of filing: 15.12.2009
(51) Int. Cl.: A61K 9/02, A61K 31/4439

(54) **Suppository comprising pantoprazole**

(71) Applicant: bene-Arzneimittel GmbH, 81479 München (DE)
(72) Inventor: Metz, Christian, 82065 Baierbrunn (DE)
(74) Representative: Peters, Hajo

(57) **Abstract**

The present invention concerns the field of pharmaceutical technology and relates to a suppository for rectal administration comprising at least one pellet, in particular, a pellet comprising the proton pump inhibitor pantoprazole. In addition, the present invention concerns a process for preparing such a suppository.

## Description

The present invention concerns the field of pharmaceutical technology and relates to a suppository for rectal administration comprising at least one pellet, in particular, a pellet comprising the proton pump inhibitor pantoprazole. In addition, the present invention concerns a process for preparing such a suppository.

Proton pump inhibitors, such as, for example, omeprazole, pantoprazole, lasoprazole etc., inhibit the gastric proton pump, i.e. the gastric H⁺, K⁺-adenosine triphosphatase (ATPase), by covalent binding to cysteine residues of the ATPase in the parietal cell (Sachs, G. et al. (2006) Review article: the clinical pharmacology of proton pump inhibitors. Curr. Gastroenterol. Rep. 10, 528-534).

However, oral administered proton pump inhibitors are metabolized through the liver via the cytochrome P450 (CYP) pathway. Therefore, plasma concentrations of co-administered drugs that are also metabolized by the CYP system, such as for example anticonvulsant, phenytoin, warfarin, might be altered (Chong, E. and Ensom, M.H. (2003) Pharmacogenetics of proton pump inhibitors: a systematic review. Pharmacotherapy 23, 460-471). In addition, genetic polymorphisms of the specific CYP2C19 might cause unpredictable variations in the clearance of the inhibitors and thereby result in increased plasma levels (Robinson, M. and Horn, J. (2003) Clinical pharmacology of proton pump inhibitors: what the practising physician need to know. Drugs 63, 2739-2754).

In contrast to most proton inhibitors (such as omeprazole and lasoprazole), the specific proton pump inhibitor pantoprazole exhibits the lowest interaction with the CYP system, and therefore, the lowest risk of side effects induced by metabolization via the CYP system. Thus, pantoprazole is one of the preferred proton pump inhibitors.

Remaining metabolization of pantoprazole via the CYP systems can be reduced by rectal administration. This non-invasive route delivers drugs via vena rectalis cranialis and vena cava inferior into the circulation. While vena rectalis craniales firstly guides the blood via vena porta to the liver and then to the circulation vena cava inferior directly guides the blood to the circulation avoiding any hepatic metabolization. Thus, rectal administration of pantoprazole offers the possibility to limit the risk of metabolization via the CYP systems and thereby results in less side effects than the above mentioned oral administration.

The rectal route has the additional advantages that proton pump inhibitors, such as pantor-prazole, can be also administered to patients in intensive care units or to patients in which oral administration is either difficult, for example in the case of hypersensitivity to taste impulse or difficulty in swallowing, or contraindicated due to vomiting or after stomach operations.

Pantoprazole, whose systematic (IUPAC) name is 5-(difluoromethoxy)-2-[[(3,4-dimethoxy-2-pyridinyl)methyl] sulfinyl]-1 H -benzimidazole, is a well-known proton pump inhibitor used for the treatment of acid-related diseases, including gastroesophageal reflux disease (GERD), Barrett's esophagus, peptic ulcer disease (PUD), Zollinger-Ellison syndrome, gastri-nomas, and esophagitis/gastritis (inflammation of the esophagus, stomach) (Robinson, M. (2005) Proton pump inhibitors: update on their role in acid-related gastrointestinal diseases. Int. J. Clin. Pract. 59, 709-715).

However, due to its molecular structure, pantoprazole exhibit a strong tendency to decompose in neutral and, in particular, acidic environment, to strongly colored decomposition products. Therefore, stability of the administration form depends on the interaction of the used pharmaceutical excipients and pantoprazole.

Typically used suppository bases are neutral to slightly acid. For instance, the commonly used hard fats are mixtures of mono-, di- and triglycerides which are obtained by esterification of fatty acids. Therefore, hydrolysis of these glycerides results in free fatty acids. The socalled acid number of a suppository base declares the amount of potassium hydroxide in milligram necessary to neutralize the free fatty acids present in one gram fat. The acids number of commonly used hard fat lies beyond 0.3. However, this number also indicates that there are enough free acids present to promote the decomposition of pantoprazole, and thereby to results in unattractive discoloration of the suppositories.

Therefore, in order to provide stable suppositories comprising pantoprazole, it is necessary to protect the acid-labile active drug from the action of the present acids within the base. Additionally, a considerable effort has to be made during preparation to avoid traces of moisture, which would promote hydrolysis of glycerides, and, which would, therefore, increase the acid number of the base.

EP0645150 is related to compositions for rectal administration comprising a proton pump inhibitor, such as a benzimidazole, and a salt of fatty acids having 6 to 20 C atoms as a stabilizer, both of which are intermingled with each other in a base for rectal administration.

EP1037607 describes suppositories comprising a plurality of individual active compound units, the acid-labile active compound in the individual active compound units being surrounded by a mixture or at least one sterol and at least one polymer, wherein the particle size of the individual units is less than 200 µm.

EP1187601 concerns administrations forms for acid-labile active compounds, such as, for example suppositories, comprising pharmaceutical excipients and multiple individual active compound units, wherein an acid-labile active compound, such as pantoprazole, is present in the individual active compound units in a matrix made of a mixture comprising at least one solid paraffin and at least one fatty alokohol or triglyceride or fatty acid ester. The multiple individual active compound units of EP1187601 are microspheres having a particle size in the range of 50 to 500µm.

In W097/34580 a suppository for acid-labile active compounds is described which, in addition to the active compound, contains poloxamer and hydrophilic natural polymers as auxiliaries.

EP0444625 discloses omeprazole compositions for rectal administration, which contain omeprazole as an active compound, a mixture of polyethylene glycols or a mixture or hard fat and sodium lauryl sulfate as well as a soluble basic amino acid.

In EP0619365 an orally administerable pellet is described, which is resistant to gastric juice, wherein the pellet consists of a core in which pantoprazole is in admixture with binder filler and optionally other auxiliary compounds, an inert water-soluble intermediate layer surrounding the core, and an outer layer which is resistant to gastric juice.

It is an object of the present invention to provide novel suppositories comprising the acid-labile active compound pantoprazole, which can be prepared without great technical effort and exhibit both good storage stability and controllability of the release of pantoprazole.

It has now been found, surprisingly, that orally administerable pellets comprising pantoprazole are suitable to be suspended in suppository base without decomposing pantoprazole, and, in addition, that a suppository comprising such pellets exhibit good stability during storage and controllability of the release of pantoprazole.

Therefore, the invention relates in a first aspect to a suppository comprising at least one pellet and suppository base, wherein the pellet comprises a core and an inert layer surrounding the core, wherein the core comprises pantoprazole.

"Pellets" according to the invention are individual active compound units comprising the active compound pantoprazole within the core of the pellet. Thereby, pantoprazole can be admixed with fillers, binders and/or other pharmaceutical auxiliaries within the core. Such pellets can be easily obtained by providing commercial available sucrose starter pellets followed by applying pantoprazole. Preferably, a solution of pantoprazole is sprayed on the sucrose starter pellets. The solution of pantoprazole might also contain any pharmaceutical auxiliary, such as, for example, a binder. Additionally, a solution of pharmaceutical auxiliaries might be also applied on the sucrose starter pellets, either before applying of pantoprazole or thereafter.

Fillers, binders and pharmaceutical auxiliaries used for the preparation of pellets are known in the art. For instance, lactose, mannitol, polyvinylpyrrolidone, microcrystalline cellulose and hydroxymethylpropylcellulose are suitable fillers, binders and pharmaceutical auxiliaries.

"Inert layers" surrounding the core of the pellets are commonly known layers usually applied before application of layers which are resistant to gastric juice. These inert layers might be either water-soluble or slightly water-soluble. For instance, an inert water-soluble layer might comprise hydroxpropylmethylcellulose and/or polyvinylpyrrolidone, optionally plasticizers, such as propylene glycol, and/or other auxiliaries such as commonly known buffers, bases or pigments. An inert slightly water-soluble film layer might comprise, for example, ethylcellulose, polyvinyl acetate, and/or inorganic pr organic materials, such as, magnesium oxide, silicon oxide or sucrose fatty acid esters.

The main advantage of using such pellets is that pellets comprising pantoprazole and an inert layer are already commercially available. Thus, preparation of the suppository according to the present invention can be performed without any great technical effort and therefore, without any high expenses.

The number of pellets within the suppository depends on the amount of pantoprazole which should be administered and the used suppository base. Preferably, one pellet comprises 5 to 30 % (w/w) pantoprazole. More preferably, one pellet comprises 5, 7.5, 10, 12.5, 15, 17.5, 20, 22.5 or 25 % (w/w) pantoprazole.

Preferably, the suppository according to the present invention contains between 1 and 400 mg pantoprazole, preferably between 5 and 80 mg of pantoprazole, more preferably between 5 and 70 mg, 5 and 60 mg, 5 and 50 mg, 5 and 40 mg. For instance, one suppository might contain 5, 10, 15, 20, 25, 30, 35, 40, 45, 50, 55, or 60 mg pantoprazole. The daily dose can be either administered as a single dose or in several doses using the suppositories of the present invention.

In one preferred embodiment of the suppository according to the present invention, the pellet further comprises a layer which is resistant to gastric juice surrounding the water-soluble layer.

Suitable layers resistant to gastric juice are known in art. For instance, a methacrylic acid/methyl methacrylate copolymer might be used, optionally, with an additionally plasticizer. Preferably, the layer resistant to gastric juice is applied on the inert water-soluble layer.

In one embodiment of the suppository of the present invention the pellet comprises pantoprazole free acid, pantoprazole salt(s), pantoprazole hydrate(s), pantoprazole cyclodextrin inclusion complex(es), amino acid salt(s) of pantoprazole with cyclodextrin, pantoprazole sodium aqua complex(es), and metal (II) complex(es) of pantoprazole, optionally in form of one of its stereoisomers, preferably enantiomers or diastereomers, its racemate or in form of a mixture of at least two of its stereoisomers, preferably enantiomers or diastereomers, or in any mixing ratio, optionally in crystalline or amorphous form.

For instance, pantoprazole free acid might be present as crystalline form III or as amorphous form.

Pantoprazole salts, such as anhydrous salts of pantoprazole, hydrate salts of pantoprazole, and hydrate solvate salts of pantoprazole are known in the art. Examples of pantopazole salts are pantoprazole sodium, pantoprazole potassium, pantoprazole zinc (II), pantoprazole magnesium, pantoprazole calcium, pantoprazole copper (II), pantoprazole mangan (II), pantoprazole cobalt (II), pantoprazole choline salt, pantoprazole tetraalkyl ammonium salt, pantoprazole sodium sesquihydrate, for example, pantoprazole sodium sesquihydrate (2:2:3) (form I), pantoprazole sodium monohydrate, for example, sodium monohydrate (form A and form B, form B is the more stable one), pantoprazole magnesium hydroxy salts having the general formula (PPI)ₓMg²⁺(OH-)_{2 x} (H20)_{z}, pantoprazole magnesium monohydrate monosolvate, wherein for instance the solvate is methyl isobutyl ketone, pantoprazole magnesium dihydrate, pantoprazole magnesium hydrate, optionally in form of one of its stereoisomers, preferably enantiomers or diastereomers, its racemate or on form of a mixture of at least two of its stereoisomers, preferably enantiomers or diastereomers, or in any mixing ratio, optionally in crystalline or amorphous form.

Crystalline forms of pantoprazole sodium salts are, for instance, II, IV, V, VI, VIII, IX, X, XI, XII, XIII, XIV, XV, XVII, XVIII, XIX, XX. Pantoprazole sodium solvates are commonly known, wherein the solvate might be acetone, butanol, methyl ethyl ketone, dimetzhylcarbonate, propanol, or 2-methylpropanol. Metal (II) complex(es) of pantoprazole are also known in the art, wherein the metal is usually Cu, Zn, Mn or Co.

Preferred enantiomers of pantoprazole are, for instance, (-)-(S)-pantoprazole and (+)- (R)-pantoprazole, (-)-(S)-pantoprazole sodium, (-)-(S)-pantoprazole potassium, (-)-(S)-pantoprazole calcium, (-)-(S)-pantoprazole magnesium, (-)-(S)-pantoprazole zinc, (-)-(S)-pantoprazole magnesium dehydrate, (-)-(S)-pantoprazole sodium solvate hydrate, (-)-(S)-pantoprazole sodium sesquihydrate, or as (-)-(S)-pantoprazole sodium monohydrate, (+)-(R)-pantoprazole sodium, (+)-(R)-pantoprazole potassium, (+)-(R)-pantoprazole calcium, (+)-(R)-pantoprazole magnesium, or (+)-(R)-pantoprazole zinc, (+)-(R)-pantoprazole magnesium dehydrate, (+)-(R)-pantoprazole sodium solvate hydrate, (+)-(R)-pantoprazole sodium sesquihydrate, or as (+)-(R)-pantoprazole sodium monohydrate. One preferred enantiomer is ()-(S)-pantoprazole, preferably as (-)-(S)-pantoprazole sodium, (-)-(S)-pantoprazole magnesium dehydrate, (-)-(S)-pantoprazole sodium solvate hydrate, (-)-(S)-pantoprazole sodium sesquihydrate, or as (-)-(S)-pantoprazole sodium monohydrate, optionally in crystalline or amorphous form.

In one particular preferred embodiment of the suppository of the present invention the pellet comprises pantoprazole as its free base, as pantoprazole sodium, as pantoprazole sodium sesquihydrate, or as pantoprazole sodium monohydrate, optionally in form of one of its stereoisomers, preferably enantiomers or diastereomers, its racemate or on form of a mixture of at least two of its stereoisomers, preferably enantiomers or diastereomers, or in any mixing ratio, optionally in crystalline or amorphous form.

In one preferred embodiment of the suppository according to the present invention, the size of the pellet is in the range of 0.1 to 1.0 mm, of 0.1 to 0.9 mm, of 0.1 to 0.8 mm, of 0.1 to 0.7 mm, of 0.1 to 0.6 mm, of 0.1 to 0.5 mm. Preferably, the size of the pellet is in the range of 0.2 to 1.0 mm, of 0.2 to 0.9 mm, of 0.2 to 0.8 mm, of 0.2 to 0.7 mm, of 0.2 to 0.6 mm, of 0.2 to 0.5 mm. More preferably, the size of the pellet is in the range of 0.25 to 1.0 mm, of 0.25 to 0.9 mm, of 0.25 to 0.8 mm, of 0.25 to 0.7 mm, of 0.25 to 0.6 mm, of 0.25 to 0.5 mm, or, alternatively, of 0.3 to 1.0 mm, of 0.3 to 0.9 mm, of 0.3 to 0.8 mm, of 0.3 to 0.7 mm, of 0.3 to 0.6 mm, of 0.3 to 0.5 mm.

In one embodiment of the suppository according to the present invention, the suppository base is selected from the group consisting of water-soluble bases, oleaginous bases, emulsion bases and ointment bases. These bases are commonly employed in the manufacture of suppositories. Water-soluble bases are, for instance, polyethylene glycol (e.g.PEG-400, 1000, 1540, 4000 and 6000, inclusive of their mixtures), glycerin, glycero-gelatin, propylene glycol, sorbitol, mannitol, aqueous gel bases such as natural gums (e.g. gum tragacanth, gum acasia, karaya gum, Irish moss, guar gum, xanthan gum, locust bean gum, etc.), cellulose derivatives (e.g. methylcellulose, carboxymethylcellulose, etc.), acrylic polymers (e.g. polyacrylic acid, polymethacrylic acid, etc.), vinyl polymers (e.g. polyvinylpyrrolidone, polyvinyl methyl ether, carboxypolymethylene, etc.), synthetic polysaccharides (e.g. poly-sucrose, polyglucose, polylactose, etc.), starch, dextrin, pectin, sodium alginate etc. Oleaginous bases are, for instance, cacao butter, laurin fat, isocacao, fatty acid glycerides such as Suppocire (Gatefosse, France), Witepsol (Dynamit Nobel, Germany)(e.g. Witepsol W-35, H-5, etc.), Migriol (Dynamit Nobel, Germany), etc., and vegetal oils such as sesame oil, soybean oil, corn oil, cottonseed oil, olive oil etc. Emulsion bases are, for instance, systems based on, for example, a) cacao buffer formulated with cholesterol and glycerin, with lecithin and water, with Lanette Wax SX (based on cetyl alcohol-stearyl alcohol sulfate ester and containing about 10% of phosphate ester), with cetyl alcohol and sodium lauryl sulfate, or with glycerin monostearate, and b) systems prepared by ading sodium lauryl sulfate, Tween, or the like to fatty acid monglycerides, monolene (propylene glycol- alpha - monostearate), wood wax, white Japan wax, stearyl alcohol, cetyl alcohol or the like. Ointment bases are, for instance, purified lanolin, sodium lauryl sulfate etc. Among others, the preferred water-soluble bases are polyethylene glycols, the preferred oleaginous bases are fatty acid mono-, di- or tri-glycerides such as Witepsols and Migriols (Dynamit Nobel, Germany), the preferred emulsion base are cacao butter-Lanette Wax SX etc., and the preferred ointment bases are purified lanolin and others. These bases are used either singly or in combination.

In one preferred embodiment of the suppository according to the present invention, the suppository base is an oleaginous base selected from the group consisting of cacao butter, laurin fat, isocacao, fatty acid glycerides and vegetable oils.

In an even more preferred embodiment of the suppository according to the present invention, the suppository base is fatty acid glycerides. Preferably, such fatty acid glycerides are hard fats used for the production of rectal suppositories. Such hard fats are also known as Adeps solidus or Adeps neutralise. Hard fats are mixtures of mono-, di- and triglycerides which are obtained by esterification of fatty acids. Such hard fats are commercially available, for instance, under the name Witepsol®. Further pharmaceutically acceptable auxiliaries, such as, stabilizers, consistency-improving additives etc. might be added.

Another aspect of the present invention relates to a process for preparing a suppository according to the present invention comprising the steps of
a) melting the suppository base,
b) cooling the suppository base,
c) suspending the at least one pellet into the suppository base while stirring constantly,
d) cooling the suspension while stirring constantly,
e) transferring the suspension into suppository forms, and
f) gradually cooling the filled suppository forms.

## Claims

1. A suppository comprising at least one pellet and suppository base, wherein the pellet comprises a core and an inert layer surrounding the core, wherein the core comprises pantoprazole.

2. Suppository according to claim 1, wherein the pellet further comprises a layer which is resistant to gastric juice surrounding the inert layer.

3. Suppository according to claim 1 or 2 comprising pantoprazole free acid, pantoprazole salt(s), pantoprazole hydrate(s), pantoprazole cyclodextrin inclusion complex(es), amino acid salt(s) of pantoprazole with cyclodextrin, pantoprazole sodium aqua complex(es), and metal (II) complex(es) of pantoprazole, optionally in form of one of its stereoisomers, preferably enantiomers or diastereomers, its racemate or in form of a mixture of at least two of its stereoisomers, preferably enantiomers or diastereomers, or in any mixing ratio, optionally in crystalline or amorphous form.

4. Suppository according to any of claims 1 to 3, wherein the size of the pellet is in the range of 0.5 to 1.0 mm, preferably in the range of 0.1 to 0.5 mm, more preferably in the range of 0.25 to 0.5 mm.

5. Suppository according to any of claims 1 to 4, wherein the suppository base is selected from the group consisting of water-soluble bases, oleaginous bases, emulsion bases and ointment bases.

6. Suppository according to claim 5, wherein the suppository base is an oleaginous base selected from the group consisting of cacao butter, laurin fat, isocacao, fatty acid glycerides and vegetable oils.

7. Suppository according to claim 6, wherein the suppository base are fatty acid glycerides.

8. Process for preparing a suppository according to any of claims 1 to 7 comprising the steps of
a) melting the suppository base,
b) cooling the suppository base,
c) suspending the at least one pellet into the suppository base while stirring constantly,
d) cooling the suspension while stirring constantly,
e) transferring the suspension into suppository forms, and
f) gradually cooling the filled suppository forms.
